# EUROPÄISCHE PATENTSCHRIFT

(11) **EP 0 675 142 B1**
(45) Veröffentlichungstag und Bekanntmachung des Hinweises auf die Patenterteilung: **27.01.1999**
(21) Anmeldenummer: 95103544.3
(22) Anmeldetag: 11.03.1995
(51) Int. Cl.: C08F 283/06, C08F 251/00, C08F 220/02, C08F 20/02, A61L 15/24, A61L 15/60, C08F 291/00

(54) **Wasserquellbare hydrophile Polymere**
Water-swellable hydrophilic polymers
Polymères hydrophiles gonflables à l'eau

(30) Priorität: 02.04.1994 DE 4411536
(43) Veröffentlichungstag der Anmeldung: 04.10.1995
(73) Patentinhaber: Clariant GmbH, 65929 Frankfurt am Main (DE)
(72) Erfinder: Funk, Rüdiger, Dr., D-65527 Niedernhausen (DE); Engelhardt, Fritz, Dr., D-60386 Frankfurt am Main (DE); Riegel, Ulrich, D-60386 Frankfurt am Main (DE); Wessling, Michael, Dr., D-63477 Maintal (DE)

(56) Entgegenhaltungen:
- EP-A- 0 545 126
- WO-A-93/19099
- WO-A-93/24153
- DE-A- 4 338 867

## Beschreibung

Die vorliegende Erfindung betrifft ein Verfahren zur Herstellung wasserquellbarer hydrophiler Polymere durch radikalische Polymerisation unter Verwendung von Tri- oder Polyradikale bildenden Radikalinitiatoren.

Wasserquellbare hydrophile Polymere, insbesondere vernetzte Polymere und Copolymere auf Acryl- oder Methacrylsäurebasis, Acrylamidopropansulfonsäurecopolymerisate oder Pfropfpolymerisate auf Stärke oder Polyalkylenoxiden sind lange bekannt und beispielsweise in US 4,931,497, US 5,011,892, US 5,041,496, US 3,926,891 und den dort zitierten Literaturstellen beschrieben.

Sie können ein Vielfaches ihres Gewichts an Wasser oder wäßrigen Flüssigkeiten, wie Urin oder Blut, aufnehmen und werden deshalb als Absorbtionsmittel insbesondere in Hygieneartikeln wie Baby- und Erwachsenenwindeln, Tampons und dergleichen eingesetzt.

Die Herstellung solcher wasserquellbarer hydrophiler Polymerer erfolgt in der Regel durch radikalische Polymerisation in wäßriger Lösung, die die Monomeren, sowie gegebenenfalls Pfropfgrundlage und Vernetzer enthält. Die Initiierung der Polymerisation kann durch energiereiche Strahlung und/oder chemisch erfolgen. Als chemische Initiatoren werden dabei beispielsweise Peroxidverbindungen, wie Peroxodisulfate, Wasserstoffperoxid, Benzoylperoxid, tert. Butylhydroperoxid, oder tert. Butylperpivalat, Azoinitiatoren wie 2,2'-Azobis(isobutyronitril) (AIBN) oder 2,2'-Azobis(2-amidinopropan)-dihydrochlorid oder Redoxsysteme wie beispielsweise Natriumperoxodisulfat/Natriumpyrosulfit oder Wasserstoffperoxid/Hydroxylaminchlorid eingesetzt. Desweiteren können Benzoin, Benzil und deren Derivate oder Acetophenonderivate als Photoinitiatoren verwendet werden. Alle diese Initiatoren haben gemeinsam, daß sie Monoradikale bilden, die die Polymerisation auslösen.

Die derart hergestellten Produkte weisen allerdings verschiedene Netzwerkdefekte auf, die auf unerwunschte Nebenreaktionen während der Polymerisation zurückgehen und die die Produkteigenschaften negativ beeinflussen. Beispielsweise werden Oligomere gebildet, die nicht in das polymere Netzwerk eingebaut sind, deshalb aus dem gequollenen Netzwerk extrahiert werden können und somit unwirksame Bestandteile sind. Daneben sind auch Polymerketten, die nur einseitig an das Netzwerk gebunden sind, unwirksam.

Es wurde nun gefunden, daß die genannten Netzwerkdefekte vermieden, bzw. weitgehend vermieden werden können, wenn als Radikalinitiatoren Verbindungen eingesetzt werden, die statt nur einer Radikalstelle pro Molekül wie die bisher gemäß Stand der Technik eingesetzten Verbindungen, drei oder mehr Radikalstellen pro Molekül ausbilden.

Die vorliegende Erfindung betrifft somit ein Verfahren zur Herstellung wasserquellbarer hydrophiler Polymere, dadurch gekennzeichnet, daß eine 15 bis 50 gew.-%ige wäßrige Lösung eines oder mehrerer hydrophiler Monomere und gegebenenfalls einer geeigneten Pfropfgrundlage nach dem Verfahren der Gelpolymerisation in Gegenwart eines Radikalinitiators, der Tri- oder Polyradikale bilden kann, polymerisiert wird.

Geeignete hydrophile Monomere sind beispielsweise polymerisationsfähige Säuren, wie Acrylsäure, Methacrylsäure, Capronsäure, Vinylsulfonsäure, Vinylphosphonsäure, Maleinsäure einschließlich dessen Anhydrid, Fumarsäure, Itaconsäure, 2-Acrylamido-2-methylpropansulfonsäure sowie deren Amide, Hydroxyalkylester und aminogruppen- oder ammoniumgruppenhaltige Ester und Amide. Desweiteren wasserlösliche N-Vinylamide oder auch Diallyldimethylammoniumchlorid.

Bevorzugte hydrophile Monomere'sind Verbindungen der allgemeinen Formel I 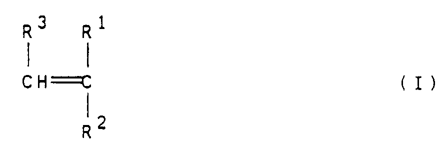 worin
- R¹: Wasserstoff, Methyl oder Ethyl,
- R²: die Gruppe -COOR⁴, die Sulfonylgruppe, die Phosphonylgruppe, die mit (C₁-C₄)-Alkanol veresterte Phosphonylgruppe oder eine Gruppe der Formel 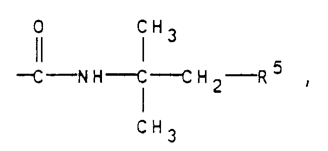
- R³: Wasserstoff, Methyl, Ethyl oder die Carboxylgruppe,
- R⁴: Wasserstoff, Amino oder Hydroxy-(C₁-C₄)-alkyl und
- R⁵: die Sulfonylgruppe, die Phosphonylgruppe oder die Carboxylgruppe
bedeuten.

Besonders bevorzugte hydrophile Monomere sind Acrylsäure und Methacrylsäure.

Geeignete Pfropfgrundlagen können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Bevorzugt sind Stärke und Polyethylen- und Polypropylenoxide, insbesondere die in US 4,931,497, US 5,011,892 und US 5,041,496 beschriebenen.

Als Radikalinitiatoren können im Prinzip alle Verbindungen eingesetzt werden, die mit oder ohne Einwirkung zusätzlicher Aktivatoren wie Licht, Strahlung, Wärme, Ultraschall, Redoxmittel usw., drei oder mehr Radikalstellen pro Molekül ausbilden. Dies bedeutet, daß diese Radikalinitiatoren drei oder mehr Gruppen enthalten, die Radikale bilden können. Die Radikalstellen können dabei gleichzeitig gebildet werden, in der Regel werden sie aber zeitlich versetzt, d.h. nacheinander gebildet. Geeignet sind beispielsweise Verbindungen, die mindestens drei Hydroperoxid-Einheiten, Peroxid-Einheiten oder Azo-Einheiten enthalten.

Geeignete Polyhydroperoxide können beispielsweise durch anodische Oxidation von Polycarbonsäuren, insbesondere von Polyacrylsäure und Polymethacrylsäure in Gegenwart von Sauerstoff erhalten werden (J. Pol. Sci. Vol. XXXIV, Seiten 287 bis 307 (1959)).

Peroxid-Einheiten können beispielsweise als Percarbonat, Perketal- oder Perester-Einheiten vorliegen. Beispiele für solche Verbindungen sind insbesondere Dioxetanverbindungen und tert.-Butylperester, wie beispielsweise Methylacrylat-tert.-Butylperacrylat-Copolymere (J. Pol. Sci. Vol. XXXIV, Seite 301 (1959)). Polymere Peroxyester können darüber hinaus durch Umsetzung von Dicarbonsäuredichloriden mit Bishydroperoxiden erhalten werden (EP-A 461 767).

Im übrigen werden in "The Chemistry of Functional Groups, Peroxides", edited by S. Patai 1983, John Wiley & Sons Ltd., Chapter 13, by Ray Ceresa geeignete Verbindungen mit mehreren Peroxid- bzw. Hydroperoxid-Einheiten und deren Synthesen beschrieben.

Es ist bevorzugt, Hydroperoxid- bzw. Peroxid-Einheiten enthaltende Radikalinitiatoren in Verbindung mit Reduktionsmitteln einzusetzen. Geeignete Reduktionsmittel sind beispielsweise Fe²⁺, Ascorbinsäure, Sulfinsäuren, Sulfite sowie Formamidinsulfinsäuren und deren Salze.

Geeignete Verbindungen, die drei oder mehr Azo-Einheiten enthalten, sind beispielsweise Umsetzungsprodukte von
a)Azodicarbonsäuren mit Verbindungen, die mehr als zwei Oxiran-Funktionen enthalten. Je nach verwendeter Oxiranverbindung können auf diese Weise Tri- bis Oligoverbindungen und Polymere erhalten werden.
   Eine bevorzugte Azodicarbonsäure ist insbesondere 4,4'-Azobis(4-cyanovaleriansäure), die beispielsweise mit Polyglycerinpolyglycidylethern geeignete Radikalinitiatoren bildet.
b) hydroxyl- und aminofunktionellen Azoverbindungen mit Verbindungen, die mehr als zwei Oxiran- oder Isocyanatgruppe enthalten.
   Geeignete Azoverbindungen sind beispielsweise 2,2'-Azobis(N,N-dimethylenisobutyramidin) oder das entsprechende Dihydrochlorid, 2,2'-Azobis(2-amidinopropan)-dihydrochlorid, 2,2'-Azobis(2-methyl-N-(1,1bis(hydroxymethyl)-2-hydroxyethyl)-propionamid), 2,2'-Azobis(2-methyl-N-(1,1-bis(hydroxymethyl)-ethyl)propionamid), oder 2,2'-Azobis(2-methyl-N-(2-hydroxyethyl)-propionamid), die beispielsweise mit den oben unter a) genannten Glycidylethern oder mit Hexamethylendiisocyanat, Toluylendiisocyanat oder Phenylendiisocyanat geeignete Radikalinitiatoren bilden.
c) Azobisamiden mit Aldehyden. Ein geeignetes Azobisamid ist insbesondere 2,2'-Azobis(isobutyramid)-dihydrat, das beispielsweise mit Formaldehyd oder Glyoxal geeignete Radikalinitiatoren bildet.
d) Azobisnitrilen mit Polyalkoholen. Bevorzugt sind insbesondere Umsetzungsprodukte von 2,2'-Azobisisobutyronitril mit Ethylenglykol, Butandiol-1,4 oder Hexandiol-1,6 (Makromol. Chem. 178 2533 (1977)).

Geeignete Initiatoren sind daneben mehrfunktionelle Photoinitiatoren, durch Umsetzung von Ce⁴⁺ mit polyfunktionellen Alkoholen, wie Polyvinylalkohol oder Cellulose erhaltene Verbindungen (J. Po. Sci. Vol. XXXI, Seite 242 f. (1958)) sowie ozonisierte Stärke (Chemistry and Engineering News, 37 27, 41 (1959)).

Die genannten Radikalinitiatoren können allein oder auch in beliebigen Mischungen untereinander im erfindungsgemäßen Verfahren verwendet werden.

Dabei werden sie bevorzugt in Mengen von 0,001 bis 20 Gew.%, bezogen auf die Gesamtmonomeren eingesetzt. Besonders bevorzugt sind 0,05 bis 3,0 Gew.%.

In einer besonderen Ausführungsform der vorliegenden Erfindung werden Radikalinitiatoren verwendet, deren Radikale bildende Funktionen unterschiedliche Reaktivitäten besitzen bzw. durch unterschiedliche Mechanismen aktiviert werden. Solche Initiatoren enthalten also beispielsweise sowohl Azo-, als auch Peroxid- oder Hydroperoxidfunktionen, die in einer vorbestimmbaren Weise nacheinander aktiviert werden und so beispielsweise zur Herstellung von Blockpolymeren verwendet werden können.

Es kann darüber hinaus von Vorteil sein, Initiatoren zu verwenden, deren radikalbildende Funktionen in unterschiedlichen räumlichen Abständen voneinander in dem Molekül liegen.

Das Molekulargewicht der im erfindungsgemäßen Verfahren verwendbaren Initiatoren kann naturgemäß in weiten Grenzen schwanken. Die Molekulargewichte liegen insbesondere im Bereich von 100 bis 10.000.000.

Das erfindungsgemäße Verfahren kann auch unter Verwendung geeigneter Vernetzer, d.h. von Verbindungen mit mindestens zwei Doppelbindungen, die in das Polymernetzwerk einpolymerisiert werden können, durchgeführt werden.

Radikalinitiatoren, die drei oder mehr Radikalstellen pro Molekül bilden, haben selbst vernetzende Eigenschaften, so daß in diesen Fällen auf die genannten Vernetzer auch verzichtet werden kann. Gleichwohl können die genannten Vernetzer auch in Kombination mit drei oder mehr Radikalstellen pro Molekül bildenden Initiatoren verwendet werden.

Geeignete Vernetzer sind insbesondere Methylenbisacryl- bzw. -methacrylamid, Ester ungesättigter Mono- oder Polycarbonsäuren von Polyolen, wie Diacrylat oder Triacrylat, z.B. Butandiol- oder Ethylenglykoldiacrylat bzw. -methacrylat, Trimethylolpropantriacrylat sowie Vinylmethacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in der EP-A 343 427 beschrieben sind.

Der Vernetzeranteil liegt bevorzugt bei 0 bis 20 Gew.%, besonders bevorzugt bei 0 bis 3 Gew.%, bezogen auf den Gesamtmonomerenanteil.

Darüber hinaus können die erfindungsgemäß hergestellten hydrophilen Polymere in an sich bekannter Weise in wäßriger Gelphase nachvernetzt oder als gemahlene und abgesiebte Polymerpartikel oberflächenvernetzt sein. Dazu geeignete Vernetzer sind Verbindungen, die mindestens zwei Gruppen enthalten, die mit den Carboxylgruppen des hydrophilen Polymeren kovalente Bindungen bilden können. Geeignete Verbindungen sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A 83 022, EP-A 543 303 und EP-A 530 438). Als Vernetzer geeignete Polyamidoamine sind insbesondere in der EP-A 349 935 beschrieben.

Das erfindungsgemäße Verfahren kann wie die bekannten Polymerisationsverfahren durchgeführt werden. Bevorzugt ist die Polymerisation in wäßriger Lösung nach dem Verfahren der sogenannten Gelpolymerisation. Dabei werden 15 bis 50 gew.%ige wäßrige Lösungen eines oder mehrerer hydrophiler Monomerer und gegebenenfalls einer geeigneten Pfropfgrundlage in Gegenwart eines Radikalinitiators, der Di- oder Polyradikale bilden kann, bevorzugt ohne mechanische Durchmischung unter Ausnutzung des Trommsdorff-Norrish-Effektes (Bios Final Rep. 363.22; Makromol. Chem. 1, 169 (1947)), polymerisiert.

Die Polymerisationsreaktion kann im Temperaturbereich zwischen 0°C und 130°C, vorzugsweise zwischen 10°C und 100°C, sowohl bei Normaldruck als auch unter erhöhtem Druck durchgeführt werden. Wie üblich kann die Polymerisation auch in einer Schutzgasatmosphäre, vorzugsweise unter Stickstoff, ausgeführt werden.

Durch mehrstündiges Nachheizen der wäßrigen Polymerisatgele im Temperaturbereich von 50 bis 130°C, vorzugsweise 70 bis 100°C, können die Qualitätseigenschaften der Polymerisate noch verbessert werden.

Die auf diesem Wege hergestellten, in Form wäßriger Gallerten vorliegenden hydrophilen Polymere können nach mechanischer Zerkleinerung mit geeigneten Apparaten durch bekannte Trocknungsverfahren in fester Form erhalten werden und zum Einsatz gelangen.

Die erfindungsgemäß hergestellten hydrophilen Polymere weisen wesentlich höhere Molekulargewichte auf, als die bekannten Polymere des Standes der Technik und weisen im Vergleich zu diesen deutliche Vorteile auf. Insbesondere haben sie ein hohes Flüssigkeitsbindevermögen bei gleichzeitig hohen Flüssigkeitsretentionswerten und hoher mechanischer Festigkeit gequollener Gelpartikel bei niedrigen extrahierbaren Anteilen.

Sie sind deshalb in hervorragender Weise als Absorptionsmittel für Wasser und wäßrige Flüssigkeiten, wie Urin oder Blut, in Hygieneartikeln wie Baby- und Erwachsenenwindeln, Binden, Tampons und dergleichen geeignet. Sie können aber auch als Bodenverbesserungsmittel in Landwirtschaft und Gartenbau, als Feuchtigkeitsbindemittel bei der Kabelummantelung sowie zum Eindicken wäßriger Abfälle verwendet werden.

### Vergleichsbeispiel 1

1,0 g (0,003571 Mol) 4,4'-Azobis(4-cyanovaleriansäure) wurden in 100 g E-Wasser bei 50°C gelöst, mit 0,337 g (0,0019379 Mol) Ethylenglykoldiglycidylether versetzt und 24 h bei Raumtemperatur stehenlassen. In einem 1 l Polymerisationskolben aus Glas wurden 400 g E-Wasser vorgelegt, 70 g (0,83 Mol) Na-Bicarbonat darin suspendiert und 200 g (2,77 Mol) Acrylsäure so zugetropft, daß ein Überschäumen vermieden wurde. Die Monomerlösung kühlte dabei auf ca. 10°C ab. Anschließend wurde die zuvor hergestellte Initiatorlösung quantitativ unter Zuhilfenahme von 50 ml E-Wasser als Nachspülmittel in den Reaktionskolben überführt und homogen verrührt. Die klare Monomerenlösung wurde jetzt ohne Rühren unter CO₂-Atmosphäre bei Raumtemperatur 14 h stehenlassen, ohne daß eine Reaktion erkennbar war. Es wurde daraufhin mit 150 ml E-Wasser verdünnt, durch N₂-Einleiten inertisiert und auf 50°C Innentemperatur angeheizt. Nach Erreichen dieser Temperatur setzte sofort die Polymerisationsreaktion ein und es entstand eine hochviskose Paste, die 12 h bei 50°C nachgetempert wurde. Eine 0,1 %ige Lösung in E-Wasser (bezogen auf Acrylsäure) des Polymeren hatte eine relative Viskosität von 28,7335, gemessen in einem Ubbelohde-Kapillarviskosimeter Typ Ic bei 25°C.

Das erhaltene Gel wurde in einem Kneter mit jeweils 44,3 Gew.% NaOH 50 %ig (bezogen auf Acrylsäure) bis zur Homogenität verknetet, anschließend mit jeweils 0,5 Gew.% (bezogen auf Acrylsäure) Methylphosphonsäurediglycidylester versetzt, bei Temperaturen von 70 bis 80°C homogenisiert und anschliessend die nach dem Austritt aus dem Kneter mechanisch zerkleinerte Masse im Luftstrom von 180°C getrocknet. Die Ware wurde gemahlen und gesiebt (850/100 µm). Es wurden folgende Performance-Daten erhalten:

| Extractables | | CRC | FSC | AUL |
|---|---|---|---|---|
| 1 h | 16 h | | | |
| [%] | [%] | [g/g] | [g/g] | [g/g] |
| 4,6 | 9,9 | 39 | 58 | 10,6 |
| CRC = Centrifuge Retention Capacity FSC = Free Swell Capacity AUL = Absorption Under Load [20 g/cm²] | | | | |

### Vergleichsbeispiel 2

1 g (0,003571 Mol) 4,4'-Azobis-4-cyanovaleriansäure wurden in 20 ml Dimethylformamid gelöst, mit 0,31 g (0,00178 Mol) Ethylenglykoldiglycidylether versetzt, auf 50°C erwärmt und 14 h bei 50°C gehalten.

In einem 1 l Polymerisationskolben wurden 600 g E-Wasser vorgelegt, 200 g Acrylsäure darin gelöst und diese Lösung unter Einleiten von N₂ auf 50°C Innentemperatur angeheizt. Daraufhin wurde die zuvor angesetzte 50°C warme Initiatorlösung quantitativ in den Reaktionskolben überführt. Nach dem Homogenisieren wurde das N₂-Einleiten beendet und die Reaktionslösung ohne Rühren stehenlassen. Die Polymerisationsreaktion setzte unmittelbar daraufhin ein, wobei eine hochviskose Paste entstand. Es wurde 12 h bei 50°C nachgeheizt. Eine 0,1 %ige Lösung in E-Wasser (bezogen auf Acrylsäure) des Polymeren hatte eine relative Viskosität von 2,5902, gemessen in einem Ubbelohde-Kapillarviskosimeter Typ Ic bei 25°C.

Das Produkt wurde einer Nachvernetzung wie in Beispiel 1 beschrieben unterzogen. Es wurden folgende Performance-Daten erhalten:

| Extractables | | CRC | FSC | AUL |
|---|---|---|---|---|
| 1 h | 16 h | | | |
| [%] | [%] | [g/g] | [g/g] | [g/g] |
| 5,8 | 10,6 | 36 | 57 | 10,3 |

### Vergleichsbeispiel 3

Vergleichsbeispiel 2 wurde wiederholt, wobei aber nur 0,155 g (0,00089 Mol) Ethylenglykoldiglycidylether eingesetzt wurden. Es wurde ein Polymer erhalten, dessen 0,1 %ige Lösung in E-Wasser (bezogen auf Acrylsäure) eine relative Viskosität von 4,2890, gemessen in einem Ubbelohde-Kapillarviskosimeter Typ Ic bei 25°C, betrug.

Das Produkt wurde einer Nachvernetzung wie in Vergleichsbeispiel 1 beschrieben unterzogen. Es wurden folgende Performance-Daten erhalten:

| Extractables | | CRC | FSC | AUL |
|---|---|---|---|---|
| 1 h | 16 h | | | |
| [%] | [%] | [g/g] | [g/g] | [g/g] |
| 5,7 | 10,3 | 35 | 58 | 9,6 |

### Vergleichsbeispiel 4

In einem gut isolierten Polymerisationskolben wurden unter adiabatischen Bedingungen zu einer Mischung aus 344 g E-Wasser, 300 g Eis aus E-Wasser und 220 g Acrylsäure unter Rühren 134,5 g NaOH 50 %ig (Neutralisationsgrad = 55 Mol-%) langsam eingerührt. Es wurde 1 g (0,45 Gew.%)Methylenbisacrylamid zugegeben, unter Rühren Stickstoff in die Lösung eingeleitet und auf 5°C eingestellt. Es wurden 0,19 g 2,5-Dimethylhexandihydroperoxid-2,5 (Produkt der PEROXID-CHEMIE GmbH, Deutschland) und anschließend 0,55 g einer 1 %igen wäßrigen Ascorbinsäurelösung zugegeben, homogen verrührt, der Rührer entfernt und unter weiterem N₂-Einleiten stehengelassen. Schon nach wenigen Minuten setzte die Reaktion ein, in deren Verlauf die Temperatur auf ein Maximum vonca. 55°C anstieg und ein schnittfestes Gel entstand. Dieses wurde unter gleichen Bedingungen ca. 6 h stehengelassen, dann mechanisch zerkleinert, in dünner Schicht im Luftstrom von 180°C getrocknet, gemahlen und gegebenenfalls gesiebt. Es wurde ein Produkt erhalten, dessen Perfomance-Daten in Tabelle I aufgeführt sind.

### Vergleichsbeispiel 5

Vergleichsbeispiel 4 wurde wiederholt, wobei aber statt 0,45 Gew.% 0,87 Gew.% Methylenbisacrylamid eingesetzt wurden. Die Performance-Daten sind Tabelle I zu entnehmen.

### Vergleichsbeispiel 6

Vergleichsbeispiel 4 wurde wiederholt, wobei aber statt 0,45 Gew.% 1,36 Gew.% Methylenbisacrylamid eingesetzt wurden. Die Performance-Daten sind Tabelle I zu entnehmen.

### Beispiel 7

Gemäß Vergleichsbeispiel 4 wurden eingesetzt:
220 g Acrylsäure mit NaHCO₃ auf einen Neutralisationsgrad von 55 Mol-% neutralisiert.
0,3 Gew.% bezogen auf Acrylsäure Trimethylolpropantriacrylat als Vernetzer.
0,068 Gew.% bezogen auf Acrylsäure des Umsetzungsproduktes von 2,2'-Azobisisobutyronitril mit Butandiol-1,4 (Pinner-Synthese analog Beispiel 1b aus Makromol. Chem. 178, 2533 (1977) als Radikalinitiator.

Zum Vergleich wurde die Synthese wiederholt, wobei aber anstelle des obengenannten Radikalinitiators lediglich die Ausgangsprodukte zu dessen Herstellung zugegeben wurden. Die Performance-Daten sind Tabelle I zu entnehmen.

### Vergleichsbeispiel 8

### a) Herstellung des Radikalinitiators:

In eine Lösung aus 600 ml wasserfreiem Cyclohexan und 140 g (1 Mol Hexamethylendiisocyanat sowie 0,3 g Zinndibutyldilaurat als Katalysator wurden unter Rühren langsam 288 g (1 Mol) 2,2'-Azobis-2-methyl-N-(2-hydroxyethyl)-propionamid eingetragen, wobei die Reaktionstemperatur durch externe Kühlung konstant bei 0 bis 10°C gehalten wurde. Es wurde 30 bis 60 min bei Raumtemperatur nachgerührt, anschließend der gebildete Niederschlag abfiltriert und unter vermindertem Druck bei maximal 40°C vom Restlösungsmittel befreit.

### b) Herstellung des Polymeren:

Gemäß Vergleichsbeispiel 4 wurden umgesetzt:
220 g einer Mischung aus Acrylsäure und Vinylphosphonsäure im Molverhältnis 100 : 1, mit NaHCO₃ auf einen Neutralisationsgrad von 55 Mol-% neutralisiert.
0,3 Gew.% bezogen auf Monomere Trimethylolpropantriacrylat als Vernetzer.
0,068 Gew.% bezogen auf Monomere des gemäß a) hergestellten Initiators.

### Vergleichsbeispiel 9

### a) Herstellung des Radikalinitiators:

Eine Lösung (schwach trüb) aus 23,6 g (0,1 Mol) 2,2'-Azo-bis-(2-methylpropionamid)-dihydrat in 500 g Wasser wurde mit verdünnter Sodalösung schwach basisch auf einen pH von 7,5 eingestellt. Anschließend wurden 14,5 g (0,1 Mol) Glyoxal 40 %ig wäßrig zugegeben, die Reaktionslösung auf 40°C erwärmt und 6 h bei dieser Temperatur gerührt. Nach Abkühlung auf < 20°C wurde die Reaktionslösung direkt für die Polymerisationsversuche eingesetzt.

### b) Herstellung des Polymeren:

Gemäß Vergleichsbeispiel 4 wurden umgesetzt:
220 g Acrylsäure, die nach der Polymerisation mit NaOH auf einen Neutralisationsgrad von 70 Mol-% neutralisiert wurde.
0,45 Gew.% bezogen auf Acrylsäure Tetraallyloxyethan als Vernetzer.
0,091 Gew.% bezogen auf Acrylsäure des gemäß a) hergestellten Initiators.

### Vergleichsbeispiel 10

Gemäß Vergleichsbeispiel 4 wurden umgesetzt:
632 g 2-Acrylamido-2-methylpropansulfonsäure, mit NaHCO₃ auf einen Neutralisationsgrad von 70 Mol-% neutralisiert.
0,1 Gew.% bezogen auf Monomer Tetraallyloxyethan als Vernetzer.
0,046 Gew.% bezogen auf Monomer des gemäß Beispiel 9a) hergestellten Initiators.

### Beispiel 11

Gemäß Vergleichsbeispiel 4 wurden umgesetzt:
220 g Acrylsäure, mit NaHCO₃ auf einen Neutralisationsgrad von 55 Mol-% neutralisiert.
0,3 Gew.% bezogen auf Acrylsäure Trimethylolpropantriacrylat.
0,022 Gew.% bezogen auf Acrylsäure 2,5-Dimethylhexandihydroperoxid-2,5 und 0,025 Gew.% bezogen auf Acrylsäure des in Beispiel 7 eingesetzten Radikalinitiators.

Zum Vergleich wurde die Synthese wiederholt, wobei aber anstelle der obengenannten Initiatormischung lediglich eine Mischung von 2,2'-Azobisisobutyronitril und Butandiol-1,4 zugegeben wurde. Die Performance-Daten sind Tabelle I zu entnehmen.

### Beispiel 12

### a) Herstellung des Radikalinitiators:

Apparatur: Doppelmantelbecherglas-Elektrolysezelle mit seitlichem Schliffansatz, Teflonstopfen mit Bohrungen für Elektroden, Gaseinleitungsrohr und Thermometer; Pt-Blech-Elektroden auf Halterung; Kryostat; Galvanostat mit Stromzuleitungen, Meßgeräten, etc.

150 g einer wäßrigen Lösung, die 8,3 % (12,45 g, 0,173 Mol-Äquiv. COOH) Polyacrylsäure (M_{w} = ca. 200000) enthielt, wurde mit 0,35 g NaOH (0,0086 Mol) versetzt, in die Elektrolysezelle überführt und mit Hilfe eines Kryostaten auf 10°C temperiert. Über das Gaseinleitungsrohr, an dessen unteres Ende eine Glasfritte angeschmolzen war, wurde nun ein steter O₂-Strom in die Lösung eingeleitet. an elektrolysierte unter Rühren bei einem Strom von 150 mA bis zu einem Ladungsdurchsatz von 1800 C, wobei die Innentemperatur bei 10°C gehalten und der Elektrolyt ständig mit Sauerstoff gespült wurde. Das Elektrolysat wurde in dieser Form unmittelbar für Polymerisationsversuche eingesetzt.

### b) Herstellung des erfindungsgemäßen Polymeren:

Gemäß Vergleichsbeispiel 4 wurden umgesetzt:
220 g Acrylsäure, die nach der Polymerisation mit NaOH auf einen Neutralisationsgrad von 68 Mol-% neutralisiert wurde.
5,8 Gew.% bezogen auf Acrylsäure des gemäß a) hergestellten Initiators.
Die Performance-Daten sind Tabelle I zu entnehmen.

Zum Vergleich wurde die Synthese wiederholt, wobei als Initiator die unter a) genannte Lösung ohne Elektrolyse Initiator die unter a) genannte Lösung ohne Elektrolyse eingesetzt wurde. Unter diesen Bedingungen fand keine Polymerisation statt (Vergleich 1).

Der Vergleich wurde wiederholt, wobei zusätzlich 0,09 Gew.% bezogen auf Acrylsäure Ammoniumperoxodisulfat zugegeben wurde. Unter diesen Bedingungen entstand ein wasserlösliches, nicht quellbares Polymer (Vergleich 2).

### Beispiel 13

Die Synthese gemäß Beispiel 12 wurde wiederholt, wobei zusätzlich 0,1 Gew.% bezogen auf Acrylsäure Methylenbisacrylamid zugegeben wurden.

Zum Vergleich wurde die Synthese wiederholt, wobei aber als Initiator die unter a) genannte Lösung ohne Elektrolyse sowie 0,09 Gew.% bezogen auf Acrylsäure Ammoniumperoxodisulfat eingesetzt wurden. Die Performance-Daten sind Tabelle I zu entnehmen.

**Tabelle I**

| Performance-Daten der Polymeren gemäß Beispielen und Vergleichsbeispielen 4 bis 13 | | | | | |
|---|---|---|---|---|---|
| Beispiel | Extractables | | CRC | FSC | AUL |
| | 1 h | 16 h | | | |
| | [%] | [%] | [g/g] | [g/g] | [g/g] |
| 4 | 4,3 | 7,4 | 38 | 54 | 9,4 |
| | | | | | |
| 5 | 1,9 | 4,5 | 28 | 47 | 21,4 |
| | | | | | |
| 6 | 1 | 2,9 | 22 | 40 | 25,3 |
| | | | | | |
| 7 | 5,1 | 8,2 | 43 | 63 | 9 |
| 7 Vergleich | 9,9 | 20 | 39 | 60 | 8,1 |
| | | | | | |
| 8 | 8,1 | 12,4 | 35 | 70 | 8,6 |
| | | | | | |
| 9 | 6,3 | 8,1 | 31 | 55 | 11,4 |
| | | | | | |
| 10 | 9,9 | 13,1 | 31 | 54 | 8,4 |
| | | | | | |
| 11 | 4,5 | 8,2 | 44 | 58 | 10 |
| 11 Vergleich | 6,2 | 9,1 | 39 | 51 | 8,8 |
| | | | | | |
| 12 | 7,6 | 16,3 | 26 | 58 | 8,3 |
| 12 Vergleich 1 | keine Polymerisation | | | | |
| 12 Vergleich 2 | wasserlösliches Polymer | | | | |
| | | | | | |
| 13 | 5,6 | 14,4 | 28 | 57 | 9,7 |
| 13 Vergleich | 7,3 | 16,9 | 25 | 56 | 8,1 |

## Patentansprüche

1. Verfahren zur Herstellung wasserquellbarer hydrophiler Polymere, dadurch gekennzeichnet, daß eine 15 bis 50 gew.-%ige wäßrige Lösung eines oder mehrerer hydrophiler Monomere und gegebenenfalls einer geeigneten Pfropfgrundlage nach dem Verfahren der Gelpolymerisation in Gegenwart eines Radikalinitiators, der Tri- oder Polyradikale bilden kann, polymerisiert wird.

2. Verfahren nach Anspruch 1, dadurch gekennzeichnet, daß die hydrophilen Monomere Verbindungen der allgemeinen Formel I 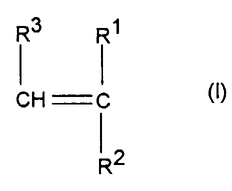 worin
R¹ Wasserstoff, Methyl oder Ethyl
R² die Gruppe -COOR⁴, die Sulfonylgruppe, die Phosphonylgruppe, die mit (C₁-C₄)-Alkanol veresterte Phosphonylgruppe oder eine Gruppe der Formel 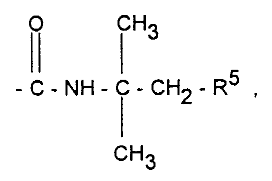
R³ Wasserstoff, Methyl, Ethyl oder die Carboxylgruppe,
R⁴ Wasserstoff, Amino oder Hydroxy-(C₁-C₄)-alkyl und
R⁵ die Sulfonylgruppe, die Phosphonylgruppe oder die Carboxylgruppe bedeuten, sind.

3. Verfahren nach Anspruch 1 und/oder 2, dadurch gekennzeichnet, daß geeignete Pfropfgrundlagen Stärke, Polyethylen- und Polypropylenoxide sind.

4. Verfahren nach einem oder mehreren der Ansprüche 1 bis 3, dadurch gekennzeichnet, daß als Radikal initiator Verbindungen eingesetzt werden, die mindestens drei Hydroperoxid-Einheiten, Peroxid-Einheiten oder Azo-Einheiten enthalten.

5. Verfahren nach einem oder mehreren der Ansprüche 1 bis 4, dadurch gekennzeichnet, daß als Radikal initiator Polyhydroperoxide eingesetzt werden, die durch anodische Oxidation von Polycarbonsäuren in Gegenwart von Sauerstoff erhalten werden.

6. Verfahren nach einem oder mehreren der Ansprüche 1 bis 4, dadurch gekennzeichnet, daß als Radikalinitiator ein Umsetzungsprodukt von 4,4'-Azobis-(4-cyanovaleriansäure) mit einem Polyglycerin-polyglycidylether eingesetzt wird.

7. Verfahren nach einem oder mehreren der Ansprüche 1 bis 6, dadurch gekennzeichnet, daß es unter Verwendung eines Vernetzers durchgeführt wird.

8. Verwendung des Verfahrens der Ansprüche 1 bis 7 zur Herstellung von wasserquellbaren hydrophilen Polymeren zur Verwendung als Absorptionsmittel für Wasser und wäßrige Flüssigkeiten in Hygieneartikeln.

## Claims

1. A process for the preparation of water-swellable hydrophilic polymers, wherein a 15 to 50% strength by weight aqueous solution of one or more hydrophilic monomers and, if required, of a suitable grafting base is polymerized by the gel polymerization method in the presence of a free radical initiator which can form triradicals or polyradicals.

2. The process as claimed in claim 1, wherein the hydrophilic monomers are compounds of the formula I 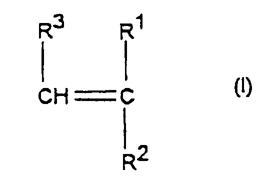 in which
R¹ is hydrogen, methyl or ethyl,
R² is the group -COOR⁴, the sulfonyl group, the phosphonyl group, the phosphonyl group esterified with (C₁-C₄)-alkanol or a group of the formula 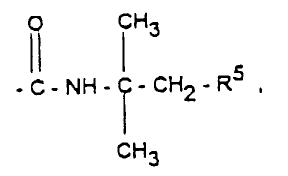
R³ is hydrogen, methyl, ethyl or the carboxyl group,
R⁴ is hydrogen, amino or hydroxy-(C₁-C₄)-alkyl and
R⁵ is the sulfonyl group, the phosphonyl group or the carboxyl group.

3. The process as claimed in claims 1 and 2 or claim 1 or 2, wherein suitable grafting bases are starch, polyethylene oxides and polypropylene oxides.

4. The process as claimed in one or more of claims 1 to 3, wherein compounds which contain at least three hydroperoxide units, peroxide units or azo units are used as the free radical initiator.

5. The process as claimed in one or more of claims 1 to 4, wherein polyhydroperoxides which are obtained by anodic oxidation of polycarboxylic acids in the presence of oxygen are used as the free radical initiator.

6. The process as claimed in one or more of claims 1 to 4, wherein a reaction product of 4,4'-azobis(4-cyanovaleric acid) with a polyglyceryl polyglycidyl ether is used as the free radical initiator.

7. The process as claimed in one or more of claims 1 to 6, which is carried out using a crosslinking agent.

8. The use of the process of claims 1 to 7 for the preparation of water-swellable hydrophilic polymers for use as absorbents for water and aqueous liquids in hygiene articles.

## Revendications

1. Procédé de préparation de polymères hydrophiles gonflables à l'eau, caractérisé en ce que l'on polymérise une solution aqueuse à 15 à 50% en poids d'un ou de plusieurs monomères hydrophiles et éventuellement d'un support de base de greffage approprié selon le procédé de la polymérisation, en gel en présence d'un initiateur radicalaire, qui peut former des tri- ou des polyradicaux.

2. Procédé selon la revendication 1, caractérisé en ce que les monomères hydrophiles préférés sont des composés de formule générale I 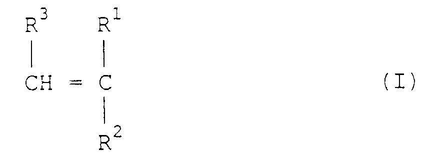 dans laquelle
R¹ représente un atome d'hydrogène, un groupe méthyle ou éthyle;
R² représente le groupe -COOR⁴, le groupe sulfonyle, le groupe phosphonyle, le groupe phosphonyle estérifié avec un alcanol en C₁-C₄ ou un groupe de formule 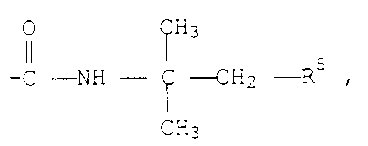
R³ représente un atome d'hydrogène, un groupe méthyle, éthyle ou le groupe carboxyle,
R⁴ représente un atome d'hydrogène, un groupe amino ou hydroxy-alkyle en C₁-C₄ et
R⁵ représente le groupe sulfonyle, le groupe phosphonyle ou le groupe carboxyle.

3. Procédé selon la revendication 1 et/ou 2, caractérisé en ce que des supports de base de greffage appropriés sont l'amidon, les polyoxydes d'éthylène et de propylène.

4. Procédé selon une ou plusieurs des revendications 1 à 3, caractérisé en ce qu'on utilise comme initiateur radicalaire des composés qui contiennent au moins trois motifs hydroperoxyde, motifs peroxyde ou motifs azoïques.

5. Procédé selon une ou plusieurs des revendications 1 à 4, caractérisé en ce qu'on utilise comme initiateur radicalaire des polyhydroperoxydes, qui sont obtenus par oxydation anodique d'acides polycarboxyliques en présence d'oxygène.

6. Procédé selon une ou plusieurs des revendications 1 à 4, caractérisé en ce qu'on utilise comme initiateur radicalaire un produit de réaction du 4,4'-azobis-(4-acide cyanovalérique) avec un polyglycidyl-polyglycérinéther.

7. Procédé selon une ou plusieurs des revendications 1 à 6, caractérisé en ce qu'il est réalisé en utilisant un agent de réticulation.

8. Utilisation du procédé selon les revendications 1 à 7 pour la préparation de polymères hydrophiles gonflables à l'eau à employer comme agent absorbant pour l'eau et les liquides aqueux dans des articles d'hygiène.
